# EUROPÄISCHE PATENTANMELDUNG

(11) **EP 0 563 501 A1**
(43) Veröffentlichungstag der Anmeldung: **06.10.1993**
(21) Anmeldenummer: 92810246.6
(22) Anmeldetag: 01.04.1992
(51) Int. Cl.: A61M 5/155

(54) **Vorrichtung zur dosierten Abgabe von Flüssigkeit und Verwendung der Vorrichtung**

(71) Anmelder: SULZER Medizinaltechnik AG, CH-8404 Winterthur (CH)
(72) Erfinder: Bittmann, Peter, Dr., CH-8057 Zürich (CH); Konaszewski, Janusz, CH-8400 Winterthur (CH); Peter, Hans, CH-8404 Winterthur (CH)
(74) Vertreter: Hammer, Bruno, Dr.

(57) **Zusammenfassung**

Bei der Dosiervorrichtung zum Dosieren von Flüssigkeit sind in einem Hohlraum (10) mit formstabilen Wänden ein erster, flexibler Behälter (11) für die zu dosierende Flüssigkeit und ein zweiter flexibler Behälter (12) der an eine Druckgasquelle angeschlossen ist angeordnet. Beim Aufblasen des Druckgasbehälters (12) wird die Flüssigkeit aus dem Flüssigkeitsbehälter (11) ausgepresst. An Stelle des flexiblen Druckgasbehälters (12), kann der Hohlraum (10), in welchem sich der Flüssigkeitsbehälter (11) liegt, gasdicht ausgeführt sein. Als Druckgasbehälter dient in diesem Fall der nicht vom Flüssigkeitsbehälter (11) ausgefüllte Teil des Hohlraums.

Das Druckgas wird beispielsweise mit einer elektrogalvanischen Druckgaszelle erzeugt, die mit einem Magneten über einen Reedschalter an- und abschaltbar ist. Die Vorrichtung gezeigte Lösung gewährleistet das einwandfreie Trennen der zu dosierenden Flüssigkeit von der Umgebung und erlaubt eine grosse Vielfalt der Formgebung für das Gehäuse und das Anpassen an den Anwender. Die Einrichtung eignet sich ausgezeichnet als Medikamentenspender aber auch als Dispenser von Schmiermitteln wie Ölen und Fetten

## Beschreibung

Die Vorliegende Erfindung bezieht sich auf eine Vorrichtung nach dem Oberbegriff von Anspruch 1 oder 2, sowie deren Verwendung.

Dosiervorrichtungen werden zur dosierten Abgabe von Flüssigkeiten und fliessfähigen Stoffen beispielsweise, von Schmiermitteln wie Ölen, Fetten aber auch Medikamenten in Lösung, verwendet. Der Ausdruck Flüssigkeit kann also im Rahmen der vorliegenden Schrift eine Flüssigkeit im eigentlichen Sinne, aber auch irgend ein fliessfähiger Stoff bedeuten. Die Dosiervorrichtungen sind geeignet und werden dazu verwendet, die Flüssigkeit kontinuierlich oder in Portionen über eine relativ lange Zeitdauer abzugeben.

Die Patentanmeldung WO 88/09187 beschreibt ein Infusionsgerät, das im wesentlichen aus einer umgebauten Injektions-Spritze besteht. Einerseits wird der Kolben der Spritze mit Druckgaszusatz aus einer galvanischen, gasentwickelnden Zelle angetrieben, d.h. vorwärts bewegt und somit die flüssige Infusion, d.h. das Medikament dosiert ausgestossen. Anderseits ist am Ausgang ein Kapillarröhrchen das als Drossel wirkt vorhanden, sodass die Infusionsflüssigkeit beim Betriebsdruck in der richtigen Menge ausgetrieben wird.

Galvanische Zellen, die Wasserstoff, bzw. Sauerstoff entwickeln, sind z.B. in DE 35.32.335 in Einzelheiten beschrieben. Selbstverständlich könnte auch irgend eine andere, nichtgalvanisch arbeitende, gasentwickelnde Zelle zum Erzeugen eines Druckgases wie CO₂, oder dergleichen, verwendet werden.

In vielen Anwendungen soll die zu dosierende Flüssigkeit, sei es ein Öl oder ein Medikament, nicht mit der Umgebung, also auch nicht mit dem Druckgas in Berührung kommen. In dieser Hinsicht bietet die Injektions-Spritze nach WO 88/09187 vor allem bei stark und schnell diffundierenden Antriebsgasen wie Wasserstoff nicht in jedem Fall ausreichenden Trenn-Schutz. Darüber hinaus ist die Form von Injektionsspritzen, vor allem für grössere Infusionsmengen von beispielsweise 20 ml oder gar 100 ml und mehr, nicht in jedem Fall vorteilhaft oder geeignet. Beispielsweise kann das Tragen eines Langzeit-Medikamentenspenders mit der Form einer Spritze und dieses Infusionsvolumens den Träger stören oder behindern. Eine andere günstigere Formgestaltung kommt deshalb kaum in Frage, weil ein Kolben mit Kreisform die bessere Dichtheit gewährleistet als jede andere, z.B. ovale oder gar eckige Kontur der Dichtung des Kolbens.

Aufgabe der Erfindung ist es, eine in dieser Hinsicht verbesserte Dosiervorrichtung zu schaffen.

Eine derartige Vorrichtung ist durch die Merkmale im Kennzeichen von Anspruch 1 oder 2 gekennzeichnet. Die abhängigen Ansprüche beziehen sich auf vorteilhafte Weiterbildungen der Erfindung.

Bei einer derartigen Vorrichtung nach der Erfindung, ist die Flüssigkeit hervorragend von der Umgebung getrennt und kann auf einfache Weise betrieben werden. Die Dosiervorrichtung ermöglicht auch, das Anpassen an verschiedenste Verwendungszwecke durch unterschiedliche Formgestaltung. Wenn der Beutel für die zu dosierende Flüssigkeit aus einem dichten Material besteht, kann jede Verunreiniung der Flüssigkeit durch diffundierende Gase oder Flüssigkeiten ausgeschaltet werden. Die zu dosierende Flüssigkeit in einem separaten und getrennten Beutel, bzw Behälter zu lagern bietet nicht nur auf einfache Weise eine verbesserte Trennsicherheit zwischen der zu dosierenden Flüssigkeit und der Umgebung bzw. dem Druckgas, sondern auch verbesserte Lecksicherheit und Überwachungsmöglichkeiten.

Die Vorrichtung kann als der Tragstelle am menschlichen oder tierischen Körper formangepasster Medikamentenspender, sei es als Wegwerf- oder mehrfachbenutzbares Gerät, besonders vorteilhaft eingesetzt, verwendet und benutzt werden. Ein derartiger, formangepasster, extrakorporaler Medikamentenspender kann beispielsweise am Thorax oder am Arm mit einem Band oder Gurt befestigt werden oder mit einer Kordel um den Hals, auf der Brust vorgehängt getragen werden.

Wenn die Vorrichtung als magnetisch oder elektromagnetisch geschalteter Langzeit-Schmiermittelspender an einer Maschine verwendet wird, kann gleichfalls die Form des Gehäuses des Schmiermittelspenders problemlos an die Ein- bzw. Anbaustelle individuell angepasst werden.

Nachstehend wird die Erfindung anhand der Figuren welche erfindungsgemäße Vorrichtungen und Teile davon schematisch zeigen, näher erläutert.

Es zeigen:
- Fig. 1: Einen Querschnitt durch eine Vorrichtung zur dosierten Abgabe von Flüssigkeit, mit einem Beutel aus flexibler Folie in einem gasdichten Hohlraum;
- Fig. 2: Einen Querschnitt durch eine Vorrichtung zur dosierten Abgabe von Flüssigkeit, mit zwei Beuteln aus flexibler Folie;
- Fig. 3: Einen elektrischen Schaltkreis für den Betrieb der galvanischen Gasquelle mit einem Magneten als Schaltelement;
- Fig. 3a: Einen Querschnitt durch eine Gasquelle mit einem elektrischen Schaltkreis mit einer Gasquelle nach Fig. 3;
- Fig. 4: In einem Querschnitt einen Beutel mit einem Einfüllstutzen der auch Port genannt wird, bei dem die Flüssigkeit mit Hohlnadeln eingefüllt werden kann;
- Fig. 4a: Einen Querschnitt durch den Einfüll- und Ausgangs-Port eines Flüssigkeitsbeutels mit einem hohlnadelartigen Ausgang;
- Fig. 4b: Die Ansicht einer Dosiervorrichtung beim Füllen des Flüssigkeitsbeutels mit einer Injektionsspritze mit Nadel;
- Fig. 4c: Die Ansicht der Dosiervorrichtung von Fig. 4a und 4b beim Aufbringen der Ausgangsschlauchs am Port;
- Fig. 5: In einem Querschnitt einen Beutel mit einem andern Einfüllstutzen für die Flüssigkeit;
Im Gehäuse 1 aus z.B. Kunststoff, befindet sich im Hohlraum 10 der Beutel 11 aus flexiblem Werkstoff, beispielsweise dreischichtiges Folienmaterial aus je einer Schicht Polyethylen innen, Aluminium in der Mitte und Polyester aussen, mit der zu dosierenden Flüssigkeit, z.B. einem Medikament. Die innere Polyethylenfolie ermöglicht das problemlose Schweissen der Beutel. Die Fuge 1' zwischen den beiden Gehäusehälften ist gasdicht. In gleicher Weise ist der Ausfluss (nicht gezeigt) des Beutels 11 gasdicht durch die Wand des Gehäuses 1 nach aussen und zum Verbraucher geführt. Wird nun im Hohlraum 10 mit Hilfe einer Druckgasquelle (nicht gezeigt) ein Überdruck erzeugt, wird die Flüssigkeit aus dem Beutel 11 gepresst. Dies geschieht unabhängig von der Form sowohl des Hohlraums 10 als auch des Beutels 11 im Hohlraum. Damit die Flüssigkeitsabgabe beim Erzeugen von Druckgas möglichst unvermittelt einsetzt, muss der Beutel 11 mit der Flüssigkeit den Hohlraum möglichst vollständig ausfüllen, bzw. der Totraum möglichst klein gehalten werden. Somit ergibt sich eine unbeschränkte Vielfalt von konstruktiven Anpassungsmöglichkeiten auch für die Gestaltung der Aussenform des Gehäuses 1.

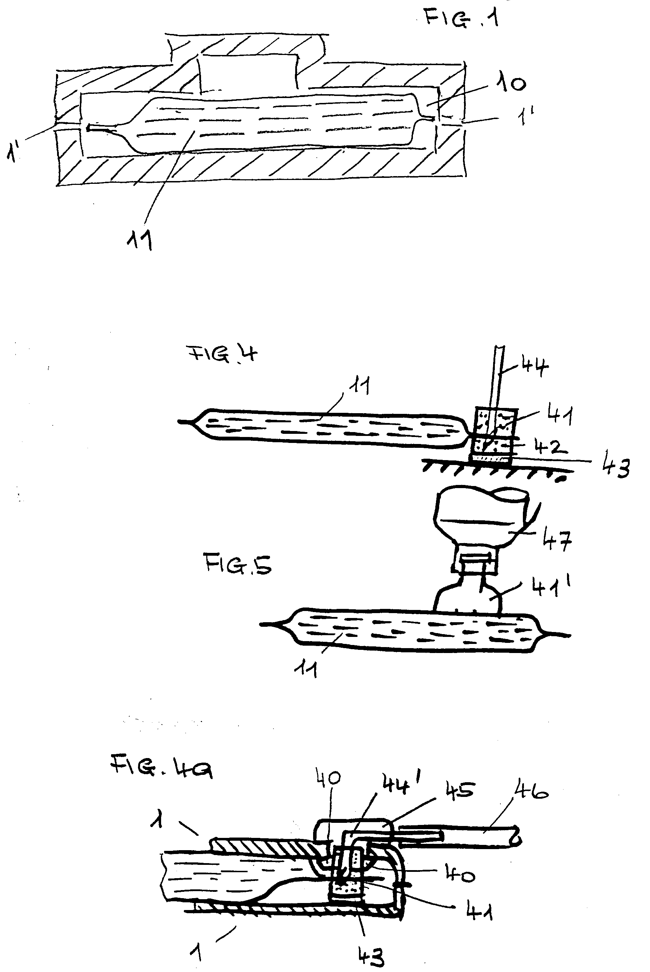

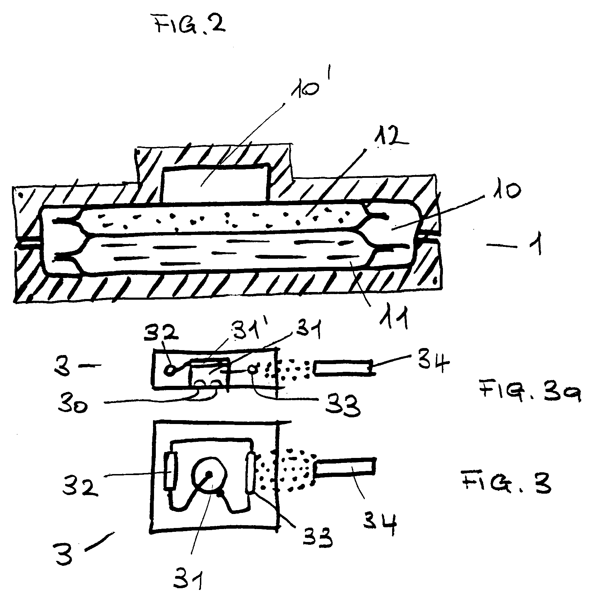

Der Hohlraum 10 der Dosiervorrichtung, von Fig. 2 braucht. im Gegensatz zu dem anhand von Fig. 1 beschriebenen Ausführungsbeispiel, nicht gasdicht zu sein. Über dem ersten Beutel 11 mit der abzugebenden Flüssigkeit liegt ein zweiter Beutel 12, der mit dem Druckgas irgend einer Druckgasquelle aufgeblasen wird und dabei die Flüssigkeit aus dem z.B. Fett-, Öl- oder Medikamentenbeutel 11 auspresst bzw. verdrängt. Um möglichst alle Flüssigkeit aus dem Beutel 11 abzugeben, sollte die Kammer des Druckgas-Beutels 12 wenigstens die gleiche Projektionsfläche wie der Flüssigkeitsbehälter im Beutel 11 aufweisen, sofern die Druckgaskammer des Gasbeutels 12 nicht aus einem elastischen, sich unter Druck ausdehnenden Werkstoff besteht. Wenn der Druckgasbeutel 12 geschweisst ist, kann in der Schweissnaht eine Schwachstelle eingebaut sein, die sich beim Überschreiten des höchsten zulässigen Drucks im Gasbeutel 12 öffnet und das Druckgas entweichen lässt.

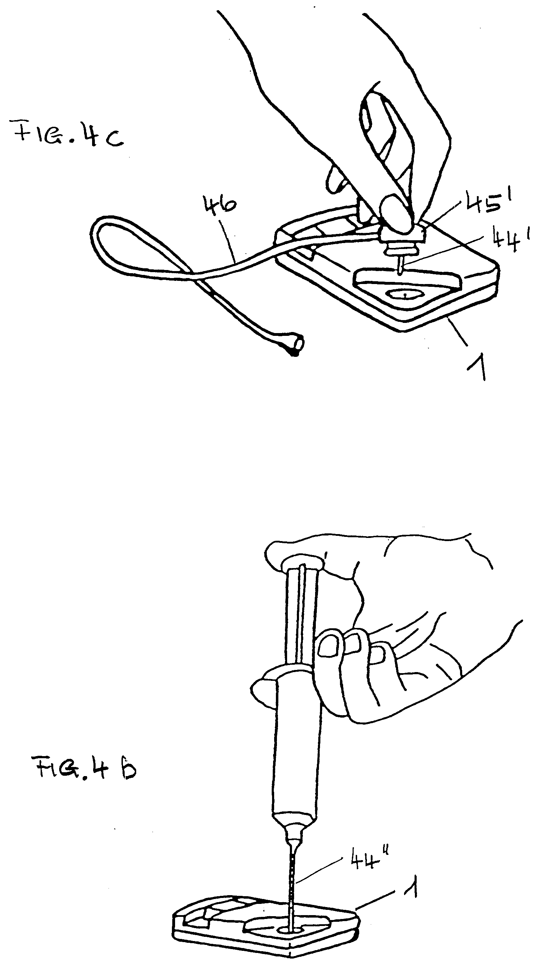

Die Druckgasquelle 31 (Fig. 3, 3a) ist mit Vorteil, zusammen mit den Elementen des Schaltkreises in einer Gussmasse eingebettet und z.B. am Druckgasbeutel so befestigt, dass das erzeugte Druckgas durch die Gasöffnungen 30 und Öffnungen in der Beutelwand in den Druckgasbeutel gelangen. Die vergossene und am Beutel 12 befestigte Druckgasquelle kommt beispielsweise in der Nische 10'(Fig. 2) des Hohlraums 10 zu liegen. Die in den Fig. 3 und 3a dargestellte Gasquelle 3 umfasst den elektrischen Schaltkreis 3 mit der galvanischen Zelle 31, in der als Stromquelle eine Batterie 31' integriert ist. Der Stromkreis mit dem Widerstand 32 weist ferner einen magnetisch betätigbaren Schalter 33, ein sogenanntes Reedrelais 33 auf. Der Widerstand 32 bestimmt die Stromstärke im Kreis und damit die pro Zeiteinheit produzierte Gasmenge der Gasquelle 31. Mit einem variablen Widerstand 32 oder mit wahlweise zuschaltbaren Widerständen kann also auch die Gasproduktion variiert werden. Beim Annähern des Permanentmagneten 34 in die ON-Position schliesst sich der Stromkreis und die galvanische Gaszelle erzeugt das Druckgas, welches nun aus den Öffnungen 30 austritt und in den Druckgasbeutel eintritt.

Um den Schalt-Zustand anzuzeigen, könnte im Stromkreis eine Lichtsignalquelle, z.B. eine Leuchtdiode vorgesehen sein, die bei eingeschalteter Dosiervorrichtung leuchtet, und welche die Funktionszustands-Überwachung erleichtert.

Es ist aber auch möglich, dass die galvanische Druckgaszelle mit dem zugehörigen Schaltkreis direkt in das Innere des Druckgasbeutels eingeschweisst ist, wodurch sich ein gasdichter Eingang für das Druckgas von aussen durch die Beutelwand oder die Schweissnaht in den Beutel erübrigt.

In Fig. 4 ist dargestellt, wie der Beutel 11 mit der dosiert abzugebenden Flüssigkeit, einem Medikament, einem Schmiermittel oder dgl. gefüllt werden kann. Im Bereich der Schweissnaht des Beutels 11 ist beidseitig ein gummiartiger Pfropfen 41, 42 angbracht. Die Nadel 44 ist in den Pfropfen 41, der als Port dient, eingestochen. Als Pfropfenmaterial ist beispielsweise Silikongummi geeignet. Die Flüssigkeit gelangt über einen Durchgang von der Einstichstelle der Nadel 44 in die Beutelwand in den eigentlichen Hohlraum des Beutels 11. Um den zu tiefen Einstich oder gar den vollständigen Durchstich der Nadel 43 durch beide Port-Pfropfen zu verhindern, kann auf der Seite, die der Einstichseite gegenüber liegt, z.B. eine Metalleinlage als sog. Nadelstop 43 vorgesehen sein. Der Port 42 könnte folienseitig, d.h. gegen den Beutel 11 hin geschlitzt sein, z.B. einen Kreuzschlitz aufweisen, was das Einfüllen der Flüssigkeit mit der Nadel 44 erleichtert.

Die Ausgangsleitung, beispielsweise eine Ausgangskanüle kann eine Drossel (nicht gezeichnet) aufweisen, welche ermöglicht, die abzugebende Flüssigkeitsmenge fein zu dosieren. Je nach Bedarf kann die Drossel gleich am Ausgang aus dem Flüssigkeitsbehälter oder sonst irgendwo in der Ausflussleitung angeordnet sein.

In der Darstellung von Fig 4a wird gezeigt, wie der Port-Pfropfen 41, 42 an einen nadelartigen Ausfluss 44' angeschlossen ist. Der abgewinkelte Ausgang 44' ist in einer knopfartigen Kappe 45 eingelassen. Die Kappe 45 kann mit einer Art Schnappverschluss oder Bajonett-Verschluss in einer Bohrung 40 am Gehäuse 1 festgemacht sein. Am abgewinkelten Ausgang 44' ist ein flexibler Katheterschlauch 46 befestigt.

Fig. 4b zeigt das Gehäuse 1 einer als Medikamentenspender ausgebildete Vorrichtung, bei der das Medikament mit der Nadel 44'' einer Injektionsspritze über den Port in den Beutel gefüllt wird. Und mit Fig. 4c wird gezeigt, wie darnach eine Katheterkappe 45' aufgesetzt wird, die als Ausfluss für den Wirkstoff dient.

Am Gehäuse kann eine Markierung und/oder eine Sollbruchstelle vorhanden sein, welche die Lage der galvanischen Druckgaszelle im Gehäuse bezeichnet. Damit wird das Entsorgen verbrauchter Druckgaszellen, z.B. mit einer Batterie als Stromquelle erleichtert, indem diese Zelle etwa mit einer Ausbruchvorrichtung aus dem Gerät ausgestanzt werden kann. Die Lage der Druckgasquelle im Gehäuse ist mit Vorteil so zu wählen, dass beim Ausstanzen, der Flüssigkeitsbeutel nicht beschädigt wird, um z.B. bei toxischen Flüssigkeiten keine Flüssigkeitsresten freizusetzen.

Fig. 5 schliesslich zeigt einen Beutel 11 mit einem anders ausgebildeten Port 41' und einem dazu passenden Anschluss-Stück 47.

In den gezeigten Beispielen weisen die Beutel 11 für die zu dosierende Flüssigkeit jeweils nur einen einzigen Port 41, 42, 43; 41' auf der als Einlass und als Auslass dient. Der Beutel 11 kann natürlich einen sparaten Auslass und Einlass aufweisen. Dies kann überall da Vorteile bringen, z.B. bei Medikamentenspendern, wo Restgasmengen im Flüssigkeitsbeutel unzulässig sind und auf einfache Art und Weise entfernt werden sollen. Wenn beispielsweise je an entgegengesetzten Enden des Flüssigkeitsbeutels die beiden Ports angebracht sind, so gelangt beim Höherstellen des Ausgangsports das Restgas unvermittelt in die Ausgangskanüle und kann so entfernt werden.

In vielen Anwendungsfällen kann es von Vorteil sein den gefüllten Flüssigkeitsbeutel in den Hohlraum des Gehäuses einzulegen, bzw. zusammen mit dem Druckgasbeutel einzugeben.

Am Gehäuse 1 kann ein Hälfte eines Klettverschlusses (Häckchen- oder Schlingenteil) aufgeklebt oder sonstwie befestigt sein, mit der die Dosiervorrichtung z.B. an einem Traggurt, welcher das Gegenstück (Schlingen oder Häckchenteil) des Klettverschusses aufweist, befestigt werden kann. Dies ist besonders bei Medikamentenspendern die vom Medikamentenempfänger ständig mitgetragen werden, eine sehr einfache, praktische Art des Befestigens.

Bei der Dosiervorrichtung zum Dosieren von Flüssigkeit sind in einem Hohlraum 10 mit formstabilen Wänden ein erster, flexibler Behälter 11 für die zu dosierende Flüssigkeit und ein zweiter flexibler Behälter 12 der an eine Druckgasquelle angeschlossen ist angeordnet. Beim Aufblasen des Druckgasbehälters 12 wird die Flüssigkeit aus dem Flüssigkeitsbehälter 11 ausgepresst. An Stelle des flexiblen Druckgasbehälters 12, kann der Hohlraum 10, in welchem sich der Flüssigkeitsbehälter 11 liegt, gasdicht ausgeführt sein. Als Druckgasbehälter dient in diesem Fall der nicht vom Flüssigkeitsbehälter 11 ausgefüllte Teil des Hohlraums.

Das Druckgas wird beispielsweise mit einer elektrogalvanischen Druckgaszelle erzeugt, die mit einem Magneten über einen Reedschalter an- und abschaltbar ist. Die Vorrichtung gezeigte Lösung gewährleistet das einwandfreie Trennen der zu dosierenden Flüssigkeit von der Umgebung und erlaubt eine grosse Vielfalt der Formgebung für das Gehäuse und das Anpassen an den Anwender. Die Einrichtung eignet sich ausgezeichnet als Medikamentenspender aber auch als Dispenser von Schmiermitteln wie Ölen und Fetten

## Patentansprüche

1. Vorrichtung zur dosierten Abgabe von Flüssigkeit aus einem Behälter (11) durch einen Auslass (41, 42, 43, 44') gekennzeichnet durch ein Gehäuse (1) mit einem formstabilen Hohlraum (10) und einen Behälter (11) aus flexiblem Material für die abzugebende Flüssigkeit in diesem Hohlraum (10) sowie eine Druckgasquelle (31), die an den Hohlraum (10) zur Abgabe von Druckgas anschliessbar oder angeschlossen ist.

2. Vorrichtung zur dosierten Abgabe von Flüssigkeit aus einem Behälter (11) durch einen Auslass (41, 42, 43, 44'), gekennzeichnet durch, ein Gehäuse (1) mit einem formstabilen Hohlraum (10) und einem erster Behälter (11) aus flexiblem Material für die abzugebende Flüssigkeit in diesem Hohlraum (10) und einem am ersten Behälter (11) anliegenden zweiten Behälter (12) aus flexiblem Material in diesem Hohlraum (10) und eine Druckgasquelle (31) die an den zweiten Behälter (12), zur Abgabe von Druckgas anschliessbar oder angeschlossen ist.

3. Vorrichtung nach Anspruch 1 oder 2, bei welcher der Behälter (11) für die abzugebende Flüssigkeit eine Einfüllvorrichtung (41, 42, 43, 44') aufweist.

4. Vorrichtung nach Anspruch 3, bei der die Einfüllvorrichtung (41, 42, 43, 44') auch der Auslass ist.

5. Vorrichtung nach Anspruch 3 oder 4, deren Einfüllvorrichtung als Einstechkörper (41, 42, 43) für eine Einfüllnadel (44) ausgebildet ist.

6. Vorrichtung nach Anspruch 5, mit einem Anschlag (43) zum Begrenzen der Eindringtiefe Einfüllnadel (44) in den Einstechkörper (41).

7. Vorrichtung nach einem der Ansprüche 1 bis 6, mit einer galvanische Zelle (31) zum Entwickeln von Druckgas als Druckgasquelle.

8. Vorrichtung nach Anspruch 7, mit magnetischen Mitteln (33, 34) zum An- und Abschalten der Stromquelle (31') der galvanischen Zelle (31).

9. Vorrichtung nach Anspruch 8, mit einem Magneten (34) zum Schalten eines Magnetschalters (33) im Stromkreis der galvanischen Zelle (31), der am Gehäuse (1) der Vorrichtung verschiebbar angebracht ist.

10. Vorrichtung nach Anspruch 2 und einem der Ansprüche 3 bis 9, bei welchem die Projektion des zweiten Behälters (12) flächengleich oder grösser als die Projektion des ersten Behälters (11) ist.

11. Vorrichtung nach Anspruch 7 bis 10, mit einer Markierung auf der Aussenseite des Gehäuses, für das Bezeichnen Lage der Stromquelle (31') für die galvanische Zelle (31) im Gehäuse (1).

12. Vorrichtung nach einem der Ansprüche 1 bis 11 bei welcher der gasdichte Hohlraum (10, Fig.1), bzw. der zweite, gasdichte Behälter (12) eine Soll-Schwachstelle aufweist, welche bei einem bestimmten Gasdruck im Behälter (12), bzw. Hohlraum (10, Fig.1), Gas entweichen lässt.

13. Vorrichtung nach einem der Ansprüche 1 bis 12, bei welcher der oder die Behälter (11, 12) aus folienartigem Werkstoff gefertigt sind.

14. Vorrichtung nach einem der Ansprüche 3 bis 13 und Anspruch 2, bei welcher der zweite Behälter (12) ein geschweisster Folienbeutel ist und eine Schweissnaht die Soll-Schwachstelle aufweist.

15. Vorrichtung nach einem der Ansprüche 1 bis 14, bei welcher der Behälter (11) für die zu dosierende Flüssigkeit zwei Ports (41, 42; 41') aufweist, die vorzugsweise an voneinander entfernten Enden angebracht sind.

16. Vorrichtung nach einem der Ansprüche 1 bis 15, bei der in der Ausgangsleitung (44; 44' 46) des Flüssigkeitsbehälters (11) eine Drossel für die zu dosierende Flüssigkeit angeordnet ist.

17. Vorrichtung nach einem der Ansprüche 3 bis 16 und Anspruch 2, wobei die Druckgsquelle (31) im Innern des Behälters (12) angeordnet ist.

18. Vorichtung nach einem der Ansprüche 1 bis 17, welche auf der Aussenseite ihres Gehäuses (1) einen Klettverschlussteil zum Befestigen an einem Gegenstück zum Klettverschlussteil aufweist.

19. Vorrichtung nach einem der Ansprüche 1 bis 18, mit einer Leuchtanzeige, welche den Schaltzustand der Dosiervorrichtung anzeigt.

20. Vorrichtung nach einem der Ansprüche 1 bis 19, mit einer Markierung und/oder Schwachstelle auf und/oder in der Wand des Gehäuses (1), welche die Lage der Druckgasquelle im Gehäuse anzeigt.

21. Verwendung einer Vorrichtung nach einem der voranstehenden Ansprüche 1 bis 20 als Medikamentenspender.
